# FASCICULE DE BREVET EUROPEEN

(11) **EP 1 641 451 B1**
(45) Date de publication et mention de la délivrance du brevet: **19.11.2008**
(21) Numéro de dépôt: 04767637.4
(22) Date de dépôt: 08.07.2004
(51) Int. Cl.: A61K 31/409, A61L 2/08

(54) **PROCEDE INACTIVATION PHOTODYNAMIQUE DES PATHOGENES AU MOYEN ALA**
VERFAHREN FÜR DIE PHOTODYNAMISCHE INAKTIVIERUNG VON PATHOGENEN MIT ALA
METHOD FOR PHOTODYNAMIC INACTIVATION OF PATHOGENS USING ALA

(30) Priorité: 09.07.2003 FR 0308424
(43) Date de publication de la demande: 05.04.2006
(73) Titulaire: MACO PHARMA, 59420 Mouvaux (FR)
(72) Inventeur: VIGNERON, Claude, F-54000 Nancy (FR); VERPOORT, Thierry, 59250 Halluin (FR); MARCHAND-ARVIER, Monique, F-74170 Les Contamines Montjoie (FR); NOTET, Véronique, F-54000 Nancy (FR); MAURICE-DUELLI, Aurore, F-54360 Blainville sur l'Eau (FR)
(74) Mandataire: Bredema
(86) Numéro de dépôt international: PCT/FR2004/001805
(87) Numéro de publication internationale: WO 2005/004862

(56) Documents cités:
- WO-A-94/06424
- WO-A-03/039606
- WO-A-03/051404
- US-A- 5 895 786
- US-B1- 6 495 098

## Description

L'invention concerne un procédé d'inactivation virale et bactérienne par traitement photodynamique in vitro d'un fluide biologique contenant des plaquettes sanguines.

Elle s'applique typiquement au cas où le fluide est un concentré plaquettaire destiné à être transfusé à un patient, par exemple soumis à une chimiothérapie ou à une intervention chirurgicale pour empêcher les hémorragies ou pour traiter des maladies comme les thrombocytopénies.

Malgré la sélection accrue des donneurs et les tests de dépistage effectués, il subsiste un risque de contamination virale (notamment avec les virus HIV-1, HIV-2, ou ceux des hépatites B et C) et bactérienne des produits sanguins labiles (PSL), notamment des concentrés plaquettaires. Le risque de contamination bactérien est d'autant plus important que les concentrés plaquettaires sont conservés à température ambiante alors que d'autres composants sanguins, tels que les concentrés de globules rouges ou le plasma, sont conservés à des températures inférieures.

De nombreuses méthodes photodynamiques ou photochimiques utilisant un agent photosensible ont été proposées pour inactiver les pathogènes, tels que les virus ou les bactéries, dans des produits sanguins.

Certaines de ces méthodes présentent l'inconvénient d'utiliser un agent photosensible, notamment synthétique, qui doit être éliminé avant la transfusion du produit sanguin au patient.

Par ailleurs, certaines méthodes connues, si elles permettent une inactivation infectieuse, ne sont en pratique pas utilisables pour inactiver les pathogènes d'un concentré plaquettaire car elles conduisent à un endommagement des plaquettes sanguines.

En particulier, le document WO-02/02153 décrit une méthode d'inactivation des pathogènes dans un fluide corporel à l'aide d'une porphyrine synthétique ou naturelle, telle que la protoporphyrine IX (PpIX). Une telle méthode, si elle est utilisée pour inactiver un concentré plaquettaire nécessite une concentration importante de protoporphyrine IX du fait d'une part que celle-ci interagit avec les protéines plasmatiques telles que l'albumine qui sont présentes en grande quantité dans un concentré plaquettaire, et d'autre part que les molécules de protoporphyrine IX possèdent des propriété physico-chimiques ne leur permettant pas d'entrer facilement dans les cellules afin d'inactiver les pathogènes intracellulaires. Il résulte de cette concentration importante que la fonctionnalité des plaquettes ne peut pas être conservée de façon suffisante à l'issue de la mise en oeuvre d'une telle méthode d'inactivation, et que si l'on diminue la concentration de protoporphyrine IX, l'inactivation n'est plus réalisée de façon satisfaisante.

Les documents WO-94/06424 et WO-97/41855 proposent des méthodes d'inactivation utilisant l'acide 5-aminolévulinique combiné avec une irradiation avec de la lumière rouge. Toutefois, les conditions d'inactivation divulguées par ces documents ne permettent pas d'obtenir une inactivation virale et bactérienne des concentrés plaquettaires in vitro qui soit suffisamment efficace.

Par ailleurs, le document WO-03/051404 décrit une méthode pour inactiver les bactéries et/ou les leucocytes dans des suspensions de thrombocytes qui prévoit d'additionner de l'acide 5-aminolévulinique puis d'appliquer un traitement photodynamique à la suspension. Cette méthode présente notamment l'inconvénient de nécessiter des temps d'incubation long (16 heures mentionnées page 13, ligne 30) avant de pouvoir réaliser le traitement photodynamique.

Il demeure donc un besoin pour obtenir une méthode d'inactivation des pathogènes, tant viraux que bactériens, dans les concentrés plaquettaires in vitro qui soit efficace et qui permette de conserver la fonctionnalité des plaquettes, et ce, tout en utilisant un précurseur d'un agent photosensible et un agent photosensible qui soient transfusables au patient. Par ailleurs, les conditions opératoires de la méthode d'inactivation doivent être compatibles avec les contraintes de productivité induite par la généralisation des traitement d'inactivation des concentrés plaquettaires.

La demanderesse a réalisé des essais intensifs et propose un procédé d'inactivation des pathogènes, notamment des virus et des bactéries d'un fluide biologique contenant des plaquettes sanguines, qui remplit l'ensemble des besoins mentionnés ci-dessus, ledit procédé comprenant les étapes prévoyant de:
- préparer et/ou disposer le fluide dans un récipient dont au moins une partie des parois est sensiblement transparente aux rayonnements de longueur d'onde comprise entre 350 nm et 450 nm ;
- introduire de l'acide 5-aminolévulinique (ALA) ou un de ses dérivés dans le fluide de sorte à permettre la transformation d'ALA en protoporphyrine IX ;
- illuminer le récipient avec au moins un rayonnement de longueur d'onde comprise entre 350 nm et 450 nm de sorte, en activant la protoporphyrine IX, à inactiver les pathogènes.
et dans lequel, préalablement à l'introduction de l'ALA, le fluide est déleucocyté.

D'autres objets et avantages de l'invention apparaîtront au cours de la description qui suit, en relation avec les figures.

La figure 1 représente le spectre d'absorption de la PpIX dans un tampon phosphate (PBS phosphate buffer saline).

Les figures 2 et 3 représentent une image obtenue par microscopie confocale de la fluorescence intraplaquettaire de la PpIX après incubation des plaquettes en présence d'une solution témoin et d'ALA.

Les figures 4 et 5 représentent l'inactivation de K. pneumoniae en présence de différentes concentrations d'ALA ou de PpIX.

Les figures 6 et 7 représentent l'inactivation de E. coli en présence d'ALA ou de PpIX, respectivement avec ou sans étape incubation.

Les figures 8 à 10 représentent des ensembles de poches pour la mise en oeuvre du procédé selon l'invention.

L'invention propose un procédé d'inactivation d'un fluide biologique contenant des plaquettes sanguines. Le procédé vise à inactiver les pathogènes présents dans le fluide, tels que des virus, bactéries, protozoaires et autres pathogènes tels que les parasites.

Le fluide biologique contenant des plaquettes est notamment un concentré plaquettaire (CP) issu d'un don de sang total ou un concentré plaquettaire obtenu par aphérèse (CPA).

Le concentré plaquettaire comprend entre 0-70% de plasma, soit 6-70 g/L de protéines plasmatiques.

Selon une réalisation particulière, le concentré plaquettaire est additionné d'une solution de conservation des plaquettes. Une telle solution est p. ex. la solution SSP (Macopharma, Tourcoing, France) dont la composition est la suivante :

| | |
|---|---|
| Citrate de sodium 2H₂O | 2,94 g |
| Acétate de sodium 3H₂O | 4,08g |
| Chlorure de sodium | 6,75 g |
| Eau injectable | 1000 mL |
| pH | 7,2 |

En particulier, le concentré plaquettaire comprend environ 30 % de plasma et 70% de solution de conservation des plaquettes de sorte à obtenir un taux de protéines de 20 g/L de protéines.

Selon l'invention, le fluide biologique contenant les plaquettes sanguines est déleucocyté préalablement à l'introduction de l'ALA dans le fluide. En effet, les leucocytes contenus dans les concentrés plaquettaires sont susceptibles de provoquer une GVHD (*Graft versus host disease,* maladie du greffon contre l'hôte) ou autres réactions immunitaires lors de la transfusion des concentrés plaquettaires. De plus, les agents pathogènes éventuellement présents dans les concentrés plaquettaires se trouvent dans un état libre ou associés aux leucocytes. Il est donc avantageux d'éliminer les leucocytes des concentrés plaquettaires.

Le fluide biologique contenant les plaquettes sanguines est préparé ou disposé dans un récipient dont au moins une partie des parois est sensiblement transparente aux rayonnements de longueur d'onde comprise entre 350 nm et 450 nm. Notamment, ce récipient est réalisé dans un matériau choisi parmi le PVC, l'EVA ou une polyoléfine telle que le polypropylène. Un tel récipient est par exemple une poche à sang.

Selon l'invention, on introduit l'acide 5-aminolévulinique (ALA) ou un de ses dérivés dans le fluide contenant les plaquettes sanguines de sorte à permettre la transformation d'ALA en protoporphyrine IX.

L'acide 5-aminolévulinique (ALA) de formule H₂N-CH₂-CO-(CH₂)₂-COOH est disponible dans le commerce (Merck, Nogent sur Marne, France). Des dérivés utiles de l'acide 5-aminolévulinique sont les esters d'ALA, en particulier les esters alkyles de l'ALA tel que l'héxyle ester de l'ALA (hALA).

Dans le procédé de l'invention, l'ALA est sous forme liquide ou solide. Sous forme solide, l'ALA est formulé par exemple sous forme de comprimé ou de poudre. Sous forme liquide, l'ALA est par exemple dilué dans un solvant tel que le NaCl ou dans une solution de conservation des plaquettes telle que la solution SSP (Macopharma, Tourcoing, France).

Selon une mise en oeuvre du procédé de l'invention, l'ALA est solubilisé préalablement à son introduction dans le fluide contenant les plaquettes, ladite solution étant introduite dans le récipient. Selon une réalisation particulière, la solubilisation est réalisée extemporanément.

Selon une variante de l'invention, l'ALA est disposé dans le récipient préalablement à la disposition du fluide dans le récipient.

Selon une autre variante, l'ALA est introduit dans le récipient lors de la disposition dudit fluide dans ledit récipient.

L'acide 5-aminolévulinique est une substance naturelle, précurseur dans la synthèse de l'hème, substance qui joue un rôle important pour la vie cellulaire et celle de l'organisme. C'est pourquoi pratiquement toutes les cellules sont capables de le synthétiser, à l'exception des globules rouges matures.

A l'intérieur des cellules, l'ALA est transformé en PpIX, substance photosensible, qui, sous l'action de la ferrochélatase, se transforme en hème. La fourniture d'ALA de façon exogène provoque une biosynthèse et une accumulation de la PpIX à l'intérieur des cellules notamment les plaquettes et les bactéries.

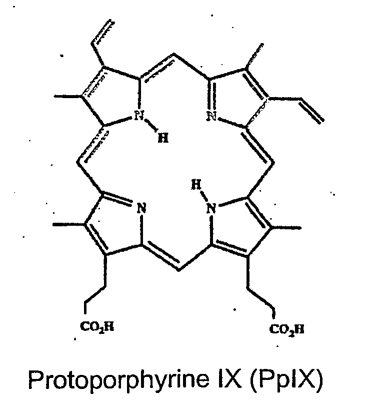

Ainsi, dans le procédé d'inactivation selon l'invention, l'ALA se transforme en PpIX de façon naturelle. Il n'est pas nécessaire de l'éliminer du concentré plaquettaire après inactivation.

La quantité d'ALA à ajouter à la suspension plaquettaire est liée à la concentration de PpIX à obtenir pour provoquer l'inactivation des pathogènes.

Cette quantité d'ALA doit également être non toxique pour les plaquettes et permettre le maintien de leur fonctionnalité. En particulier, la concentration d'ALA dans le fluide contenant les plaquettes est inférieure ou égale à 5 mM.

En outre, le plasma contient une grande quantité de fer (environ 23 +/- 11 µM/L). Or le fer se complexe avec la PpIX pour former l'hème. En ajoutant un agent chélateur de fer au fluide, la conversion de la PpIX en hème est inhibée et l'accumulation de PpIX dans les cellules et le milieu est favorisée. Selon un aspect particulier le procédé comprend, préalablement à l'illumination, l'introduction dans le fluide d'un chélateur de fer.

Des agents chélateurs appropriés sont, par exemple, la desferrioxamine, l'EDTA, CDTA (*cyclohexane diamine tetraacetic acid*), le DTPA ou le DOTA.

Selon une mise en oeuvre particulière du procédé d'inactivation, préalablement à l'illumination, le fluide additionné d'ALA est incubé pendant un temps compris entre 0 et 5 heures, en particulier entre 30 minutes et 1 h30.

L'introduction et éventuellement l'incubation d'ALA dans le fluide sont réalisées entre 0 et 40°C, en particulier à 37°C ou dans une gamme de température définie entre 20 et 24°C qui représente la gamme de température de conservation des plaquettes.

Après avoir introduit l'ALA dans le fluide, le récipient contenant le fluide est illuminé avec au moins un rayonnement de longueur d'onde comprise entre 350 et 450 nm de sorte, en activant la PpIX, à inactiver les virus et les bactéries.

La PpIX sous l'action de la lumière, subit une réaction d'oxydation et agit comme un photosensibilisant. Il y a alors production d'oxygène singulet qui réagit avec les composants des cellules ou produit des radicaux susceptibles d'endommager les cellules.

La PpIX est également transportée en dehors des cellules et peut contribuer à l'inactivation des contaminants libres comme les virus ou les bactéries extracellulaires.

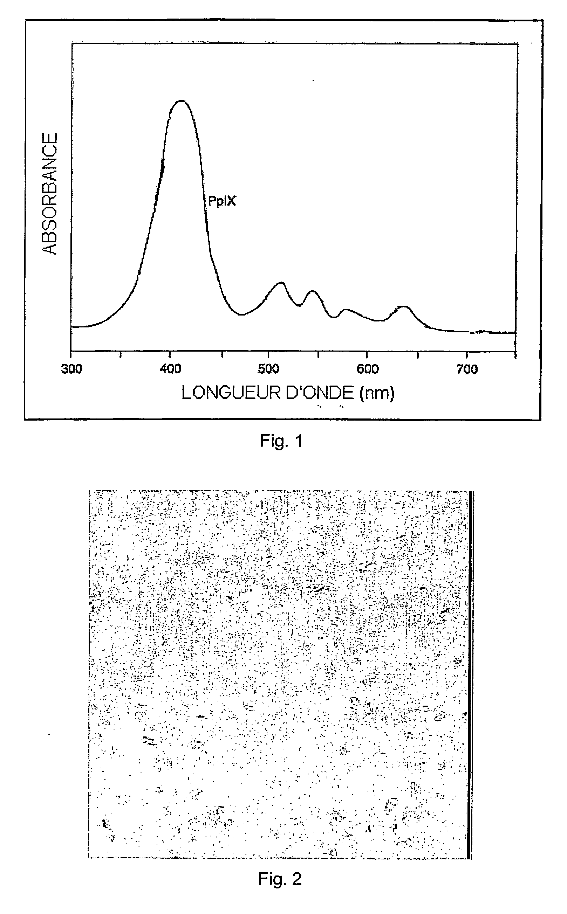

En particulier, l'illumination se fait avec un rayonnement de longueur d'onde comprise dans la bande de Soret (390-430 nm) et/ou égale à 365 nm (cf. Fig.1).

L'illumination est réalisée pendant un temps compris entre 30 minutes et 2 heures avec une énergie fournie comprise entre 10 J/cm² et 100 J/cm².

Selon une mise en oeuvre particulière, l'illumination est fractionnée, c'est-à-dire des phases d'illumination alternent avec des phase d'incubation à l'obscurité, la durée totale d'illumination restant comprise entre 30 minutes et 2 heures.

Le procédé d'inactivation selon l'invention est réalisé à une température comprise entre 0°C et 40°C. Dans une mise en oeuvre particulière, la température est contrôlée à la température de conservation des plaquettes, à savoir entre 20 et 24°C.

Pour une meilleure répartition du photosensibilisant et de l'illumination et pour une meilleure conservation des plaquettes, les récipients contenant le fluide contenant les plaquettes sont agités pendant l'illumination.

### EXEMPLES

### Matériel et méthode

### Préparation du concentré plaquettaire d'aphérèse (CPA) :

Le sang est prélevé sur des donneurs volontaires sains. Les plaquettes sont ensuite collectées sur MCS+ (Haemonetics, Baintree, MA, USA) en présence d'anticoagulant AM 16 (acide citrique 12,6 g, citrate de sodium 35,6 g, dextrose 51,64 g). Huit cycles de centrifugation (4800 RPM) permettent la séparation du concentré plaquettaire du sang total. Puis plasma et plaquettes sont transférées dans une poche après avoir subi une filtration pour éliminer les leucocytes.

La concentration en protéines du CPA est ajustée de la manière suivante :
- centrifugation du CPA à 2800T/min à 21 °C durant 15 minutes,
- prélèvement du volume de plasma de telle façon qu'il ne reste plus que la quantité voulue en protéines, et
- remise en suspension du culot de plaquettes avec une solution de conservation des plaquettes (SSP) pour obtenir la concentration voulue en protéines.
Une numération plaquettaire est effectuée sur un échantillon du CPA ajusté en protéines (Advia, Bayer).

### Les produits :

Sauf indication contraire, le 5-ALA (Merck, Nogent sur Marne, France) est dilué extemporanément dans une solution de NaCl 0,9%.

La protoporphyrine IX est sous forme de PpIX sel disodique de PM=602,62 (Aldrich, réf 10029MO). La PpIX est solubilisée dans l'eau de façon à obtenir les concentrations voulues et conservée à l'abri de la lumière.

### Méthode de contamination :

Les bactéries sont injectées dans les échantillons à une concentration finale de 1 bactérie pour 50 000 plaquettes.
Les bactéries sont mises en culture dans un bouillon Thyoglycolate-Resazurine (ref: 42074, Biomérieux Marcy l'Etoile, France) (0,5 mL de suspension bactérienne en bouillon ou une colonie sur gélose dans 9 mL de bouillon). Elles sont incubées 24 h à 37°C pour qu'elles soient en phase de croissance lors de leur utilisation. Avant leur utilisation, le bouillon de culture est centrifugé à 2500 g pendant 10 min. à 21°C, le culot est repris dans du NaCl 0,9 % et celui-ci est lavé 3 fois à 2500 g pendant 10 min. à 21 °C.

Le titre bactérien de la suspension lavée et diluée est évalué par une lecture de densité optique à 630 nm. Une dilution de la suspension bactérienne est ensuite réalisée pour obtenir une concentration finale de 1 bactérie pour 50 000 plaquettes.

### Test de bactério-atténuation :

Pour réaliser les tests de bactério-atténuation, les CPA dilués sont aliquotés dans la solution SSP (MacoPharma, Tourcoing, France) pour obtenir une concentration finale en protéines de 10 g/L dans des poches en PVC. Puis 1 mL de suspension bactérienne est injecté. Le volume final contenu dans les poches est de 8 mL. Le tout est homogénéisé pour un meilleur contact entre les plaquettes et les bactéries.

L'illumination se fait à l'aide d'une lampe UVA comportant deux tubes (réf. VL-215BL, Vilber Lourmat, France) génératrice de rayons UV 365 nm et de lumière visible d'une intensité de 108 µW/cm² à une distance de 1 m de l'échantillon. La lampe est disposée à une distance de 13 cm des poches à traitées. L'illumination se fait à 4°C. La lampe délivre une dose de 23 J/cm² en 1 h.

Après illumination, une numération bactérienne est réalisée pour mesurer la diminution du tire bactérien dans les poches traitées. Pour cela, 1 mL de la dilution la plus appropriée est étalée sur des géloses nutritives Trypcase-soja (ref : 43011, boîtes de 90 mm de diamètre, Biomérieux, Marcy l'Etoile, France). Les géloses sont ensuite incubées 24 h à 37 °C pour pouvoir effectuer un comptage. Les résultats sont exprimés en ufc/mL.

### Exemple 1: Production de PpIX intraplaquettaire à partir d'ALA visualisée en microscopie confocale

Les essais ont été réalisés sur des CPA prélevés sur un anticoagulant non auto-fluorescent : le citrate.

Les plaquettes sont incubées 1 h en présence d'ALA 32 mM dans du PBS (concentration finale de 8 mM dans les poches de CPA) ou d'une solution témoin de PBS (contrôle négatif). Après une heure d'incubation, les plaquettes sont fixées par du glutaraldéhyde 1% (1 volume d'échantillon + 3 volumes de glutaraldéhyde).

L'image de la figure 2 montre les contrôles négatifs présentant une légère autofluorescence des plaquettes.

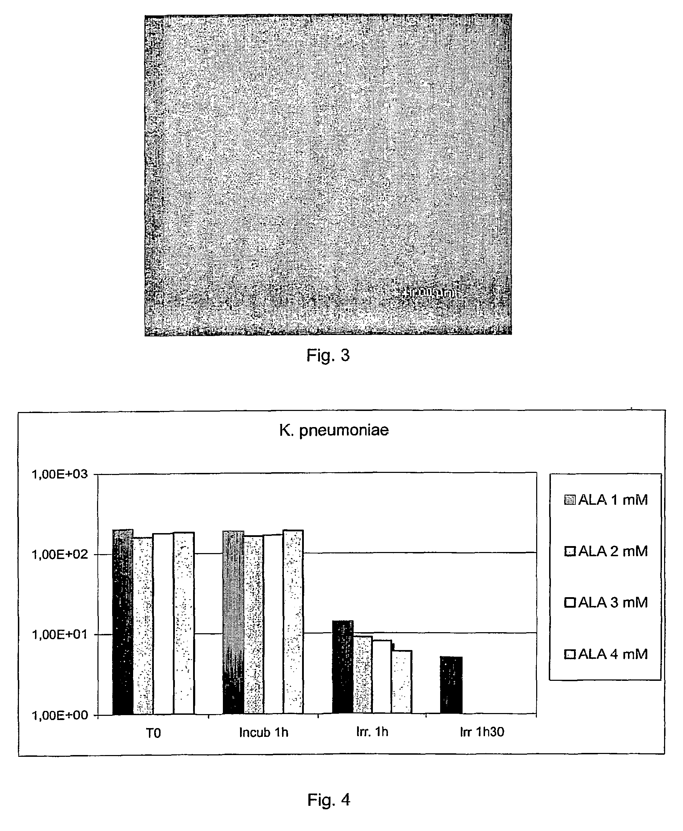

L'image de la figure 3 montre la présence de PpIX intraplaquettaire lors de l'incubation des plaquettes avec de l'ALA.

Ce test confirme qu'il est possible d'obtenir de la PpIX endogène à partir d'ALA dans un concentré plaquettaire.

### Exemple 2: Fonctionnalité des plaquettes en présence d'ALA

Dans cet exemple, deux tests ont été effectués afin d'évaluer la fonctionnalité des plaquettes en présence de différentes concentrations d'ALA après illumination à l'aide des lampes UVA (365 nm).

Le premier test est la visualisation du tournoiement ou « swirling », c'est-à-dire l'inspection visuelle du CPA à travers une source lumineuse (phénomène lié à la présence de formes discoïdes dans la suspension plaquettaire).

Le second test est un test d'agrégation au collagène. Ce test est réalisé à l'aide d'un thrombo-agrégomètre (SEFAM, Regulest, Fleurange, France). L'activation plaquettaire par un agoniste (collagène) est estimée in vitro par turbidimétrie, en enregistrant en continu à 37°C et sous agitation, la transmission d'un faisceau de lumière à travers la suspension plaquettaire. Le regroupement des plaquettes en amas de plus en plus volumineux s'accompagne d'un éclaircissement progressif du milieu. Les résultats sont rendus en pourcentage d'agrégation en fonction du temps, le 100% d'agrégation correspondant au plasma pauvre en plaquettes et le 0% d'agrégation correspondant à l'échantillon avant l'ajout de l'agoniste. L'enregistrement de la transmission lumineuse est déclenché au moment de l'ajout d'un faible volume de l'agoniste à l'échantillon. Ainsi, nous pouvons visualiser le temps de latence, le changement de forme des plaquettes puis l'agrégation qui peut être stable ou réversible.

Ces deux tests ont été réalisés sur un CPA ajusté à 10 g/L de protéines en présence de concentration croissante d'ALA après 1 h30 d'illumination à 365 nm.

| | ALA 1 mM | ALA 2 mM | ALA 3 mM | ALA 4 mM | ALA 5 mM | eau |
|---|---|---|---|---|---|---|
| Swirling | + | + | + | Faible+ | - | + |
| % d'aggrégation | 80 | 69 | 76 | 53 | 26 | 79 |

Le même test a été réalisé sans illumination (1 h30 d'agitation douce à l'obscurité)

| | ALA 1 mM | ALA 2 mM | ALA 3 mM | ALA 4 mM | ALA 5 mM | eau |
|---|---|---|---|---|---|---|
| Swirling | + | + | + | + | + | + |
| % d'aggrégation | 84 | 84 | 82 | 76 | 78 | 78 |

L'illumination d'un CPA en présence d'une concentration trop élevée d'ALA semble faire perdre de leur fonctionnalité aux plaquettes.

### Exemple 2 comparatif : fonctionnalité des plaquettes en présence de PpIX

Un test d'agrégation et une inspection visuelle du tournoiement ont été réalisées sur un CPA ajusté à 10 g/L de protéines en présence de PpX sel disodique dans du NaOH 1 N 0,1 % (après 30 minutes d'illumination à 365 nm). Le nombre de plaquettes par poche de 8 ml est de 7,6.10⁹.

| | | | | | | |
|---|---|---|---|---|---|---|
| | PpIX 20 µM | PpIX 10 µM | PpIX 5µM | PpIX 1 µM | NaOH | CPA |
| Swirling | - | - | - | + | + | + |
| % d'aggrégation | 15 | 16 | 16 | 53 | 56 | 59 |

Le swirling des plaquettes reste positif lorsque celles-ci sont irradiées à 365 nm en présence d'un concentration de PpIX inférieure à 1 µM. Au dessus de cette concentration, la fonctionnalité des plaquettes n'est pas conservée.

### Exemple 3 : inactivation de Klebsiella pneumoniae

Des tests de bactério-atténuation ont été effectués sur Klebsiella pneumoniae. Les tests sont réalisés sur des CPA ajustés à 10 g/L de protéines. Le nombre de plaquettes par poche de 8 mL est de 10,5.10⁹.

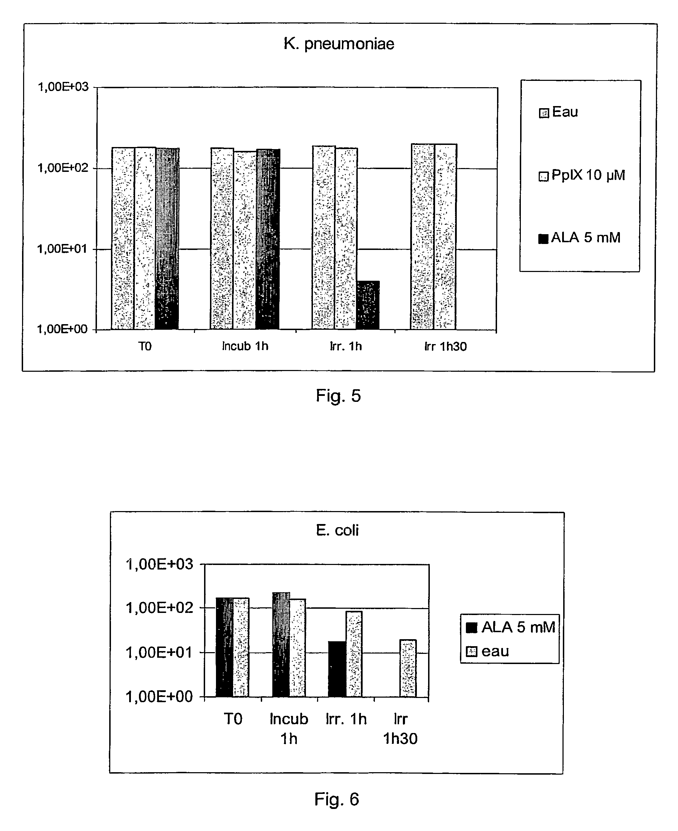

Les poches sont incubées 1h à température ambiante puis illuminées par irradiation à 365 nm (Fig. 4 et 5).

| Klebsiella | ALA 1 mM | | | | ALA 2 mM | | | | ALA 3 mM | | | |
|---|---|---|---|---|---|---|---|---|---|---|---|---|
| | T0 | Incub. 1 h | Irr. 1 h | Irr. 1h30 | T0 | Incub 1 h | Irr. 1 h | Irr 1 h30 | T0 | Incub 1 h | Irr. 1 h | Irr 1 h30 |
| 10⁻² ufc/ml | 201 | 187 | 14 | 5 | 159 | 163 | 9 | 1 | 179 | 168 | 8 | 1 |

| Klebsiella | ALA 4 mM | | | | ALA 5 mM | | | | eau | | | |
|---|---|---|---|---|---|---|---|---|---|---|---|---|
| | T0 | Incub 1h | Irr. 1h | Irr 1h30 | T0 | Incub. 1h | Irr. | Irr 1h 1h30 | T0 | Incub 1h | Irr. 1h | Irr 1h30 |
| 10⁻² ufc/ml | 183 | 192 | 6 | 1 | 178 | 175 | 4 | 0 | 185 | 180 | 187 | 199 |

| Klebsiella | PpIX 10 µM | | | |
|---|---|---|---|---|
| | T0 | Incub 1h | Irr. 1h | Irr 1h30 |
| 10⁻² ufc/ml | 181 | 162 | 178 | 203 |

Dans les poches témoins n'ayant pas subi d'illumination, le titre bactérien ne diminue pas.

Il résulte de ce test que l'inactivation de K. pneumoniae est obtenue pour des CPA contenus dans des poches irradiées 1h et 1 h30 à 365 nm en présence d'ALA, sans différence significative entre les différentes concentrations.

Par contre, dans les mêmes conditions, la PpIX 10µM ne permet pas l'inactivation.

### Exemple 4 : inactivation de Escherichia coli.

Des tests de bactério-atténuation ont été effectués sur E. coli. Les tests sont réalisés sur des CPA ajustés à 10 g/L de protéines. Le nombre de plaquettes par poche de 8 ml est de 4,7 10⁹.

Les poches sont incubées 1 h à 37°C avant d'être irradiées à 365 nm (Fig. 6).

| E. coli | ALA 5 mM | | | | Eau | | | |
|---|---|---|---|---|---|---|---|---|
| | T0 | Incub 1h | Irr. 1h | Irr 1h30 | T0 | Incub 1h | Irr. 1h | Irr 1h30 |
| 10⁻² ufc/ml | 172 | 231 | 17 | 1 | 174 | 162 | 87 | 20 |

L'inactivation de E. coli est obtenue pour des CPA contenus dans les poches irradiées 1 h et 1 h30 à 365 nm en présence d'ALA.

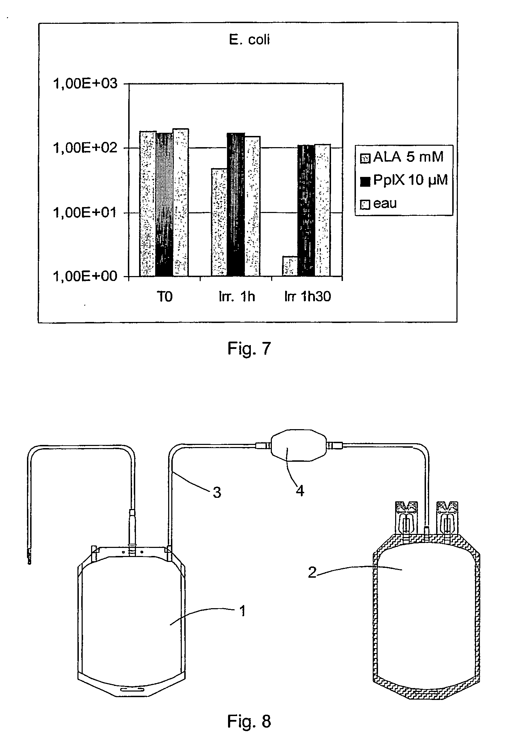

Sans incubation, les résultats sont les suivants (Fig. 7) :

| E. Coli | ALA 5 mM | | | PpIX 10 µm | | | Eau | | |
|---|---|---|---|---|---|---|---|---|---|
| | T0 | Irr. 1h | Irr 1 h30 | T0 | Irr. 1h | Irr 1 h30 | T0 | Irr. 1h | Irr 1 h30 |
| 10⁻² ufc/ml | 183 | 48 | 2 | 166 | 171 | 108 | 197 | 153 | 115 |

Alors que la PpIX est inefficace à inactiver E.coli à une concentration de 10 µM, l'ALA, même sans incubation est efficace.

La méthode d'inactivation selon l'invention a prouvé son efficacité sur les phatogènes bactériens, comme illustré dans les exemples donnés ci-dessus, mais également pour l'inactivation des virus et autres pathogènes.

En relation avec les figures 8 à 10, on décrit des systèmes à poches qui sont adaptés pour mettre en oeuvre le procédé d'inactivation de l'invention. Les poches, tubulures et récipients sont connectés à l'aide d'un appareil de connexion stérile au moment de leur utilisation ou bien les connexions sont réalisées au moment de la fabrication.

Une poche 1 contenant un concentré plaquettaire est en communication fluidique à une poche 2 en EVA par l'intermédiaire d'une tubulure 3.

Selon une première variante (non représentée), l'ALA est contenu dans la poche 2 en EVA. Lors de la mise en oeuvre du procédé d'inactivation, le concentré plaquettaire, éventuellement additionné de solution de conservation des plaquettes et contenu dans la poche 1 est envoyé dans le poche 2 en EVA.

Selon une deuxième variante représentée sur la figure 8, la tubulure 3 comprend un récipient 4 contenant l'ALA. Lors du transfert du concentré plaquettaire contenu dans la poche 1 vers la poche 2 en EVA, l'ALA est introduit dans le concentré plaquettaire.

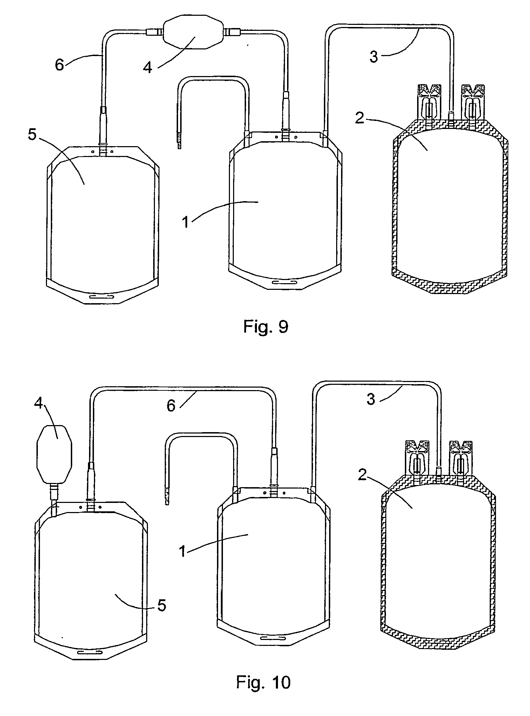

Selon une troisième variante représentée sur la figure 9, la poche 1 contenant le concentré plaquettaire est en outre connectée à une poche 5 contenant une solution de conservation des plaquettes par l'intermédiaire d'une tubulure 6 sur laquelle est disposé un récipient 4 contenant l'ALA. Dans un premier temps, la solution de conservation des plaquettes est transférée de la poche 5 vers la poche 1 contenant le concentré plaquettaire en solubilisant ou diluant l'ALA contenu dans le récipient 4. Puis le mélange concentré plaquettaire, ALA et solution de conservation des plaquettes est envoyé dans le poche 2 en EVA.

Dans une quatrième variante représentée sur la figure 10, la poche 1 contenant le concentré plaquettaire est connectée à une poche 5 contenant une solution de conservation des plaquettes par l'intermédiaire d'une tubulure 6. Le récipient 4 contenant l'ALA, notamment un flacon en verre, est placé en communication fluidique avec la poche 5 contenant une solution de conservation des plaquettes. Dans cette variante, l'ALA est d'abord introduit dans la solution de conservation des plaquettes. Le mélange ALA et solution de conservation des plaquettes est envoyé dans la poche 1 contenant le concentré plaquettaire. Puis le mélange comprenant le concentré plaquettaire, l'ALA et la solution de conservation des plaquettes est finalement envoyé dans la poche 2 en EVA.

Selon la dernière étape du procédé d'inactivation, la poche 2 en EVA est illuminée avec un rayonnement de longueur d'onde comprise entre 350 et 450 nm.

## Revendications

1. Procédé d'inactivation des pathogènes, notamment des virus et bactéries d'un fluide biologique contenant des plaquettes sanguines, ledit procédé comprenant les étapes prévoyant de :
- préparer et/ou disposer le fluide dans un récipient dont au moins une partie des parois est sensiblement transparente aux rayonnements de longueur d'onde comprise entre 350 nm et 450 nm ;
- introduire de l'acide 5-aminolévulinique (ALA) ou un de ses dérivés dans le fluide de sorte à permettre la transformation d'ALA en protoporphyrine IX ;
- illuminer le récipient avec au moins un rayonnement de longueur d'onde comprise entre 350 nm et 450 nm de sorte, en activant la protoporphyrine IX, à inactiver les pathogènes ;
ledit procédé étant **caractérisé en ce que**, préalablement à l'introduction de l'ALA, le fluide est déleucocyté.

2. Procédé selon la revendication 1, **caractérisé en ce que** le fluide biologique est un concentré plaquettaire.

3. Procédé selon la revendication 1 ou 2, **caractérisé en ce que** l'ALA est solubilisé préalablement à son introduction dans le fluide, ladite solution étant introduite dans le récipient.

4. Procédé selon l'une quelconque des revendications 1 à 3, **caractérisé en ce que** l'ALA est disposé dans le récipient préalablement à la disposition du fluide dans ledit récipient.

5. Procédé selon l'une quelconque des revendications 1 à 3, **caractérisé en ce que** l'ALA est introduit dans le fluide lors de la disposition dudit fluide dans le récipient.

6. Procédé selon l'une quelconque des revendications 1 à 5, **caractérisé en ce que** la concentration d'ALA introduite dans le fluide est inférieure ou égale à 5 mM.

7. Procédé selon l'une quelconque des revendications 1 à 6, **caractérisé en ce que**, préalablement à l'illumination, on introduit en outre dans le fluide un chélateur de fer.

8. Procédé selon l'une quelconque des revendications 1 à 7, **caractérisé en ce que**, préalablement à l'illumination, le fluide additionné d'ALA est incubé pendant un temps compris entre 0 et 5 heures.

9. Procédé selon l'une quelconque des revendications 1 à 8, **caractérisé en ce qu'**il est réalisé à une température comprise entre 0°C et 40°C.

10. Procédé selon l'une quelconque des revendications 1 à 9, **caractérisé en ce que** l'illumination se fait avec un rayonnement de longueur d'onde compris dans la bande de Soret et/ou égale à 365 nm.

11. Procédé selon l'une quelconque des revendications 1 à 10, **caractérisé en ce que** l'illumination est réalisée pendant un temps compris entre 30 minutes et 2 heures avec une énergie fournie comprise entre 10 J/cm² et 100 J/cm².

## Claims

1. A method for inactivating pathogens, more particularly viruses and bacteria in a biological fluid containing blood cells, said method comprising the steps consisting in:
- preparing and/or placing the fluid in a container, at least a part of the walls of which are substantially transparent to radiations with a wavelength between 350nm and 450nm;
- introducing 5-aminolevulinic acid (ALA) or a derivative thereof into the fluid so as to allow the transformation of the ALA into protoporphyrin IX,
- irradiating the container with at least one radiation with a wavelength between 350nm and 450nm so that, upon the activation of the protoporphyrin IX, the pathogens are inactivated;
said method being **characterized in that** the leukocytes are removed from the fluid, prior to the introduction of the ALA.

2. A method accord to claim 1, **characterized in that** the biologic fluid is a blood cells concentrate.

3. A method according to claim 1 or 2, **characterized in that** the ALA is solubilized prior to being introduced into the fluid, with said solution being introduced into the container.

4. A method according to any one of claims 1 to 3, **characterized in that** the ALA is placed in the container prior to the placing of said fluid in said container.

5. A method according to any one of claims 1 to 3, **characterized in that** the ALA is introduced into the fluid upon the placing of said fluid in the container.

6. A method according to any one of claims 1 to 5, **characterized in that** the concentration of ALA introduced into the fluid is lower than or equal to 5mM.

7. A method according to any one of claims 1 to 6, **characterized in that**, prior to the irradiation, an iron chelating agent is further introduced into the fluid.

8. A method according to any one of claims 1 to 7, **characterized in that**, prior to the irradiation, the ALA added fluid is incubated for a time between 0 and 5 hours.

9. A method according to any one of claims 1 to 8, **characterized in that** it is carried out at a temperature between 0°C and 40°C.

10. A method according to any one of claims 1 to 9, **characterized in that** the irradiation is carried out with a radiation having a wavelength within the Soret band and/or equal to 365nm.

11. A method according to any one of claims 1 to 10, **characterized in that** the irradiation is carried out for a time between 30 minutes and 2 hours, with the supplied energy being between 10J/cm² and 100J/cm².

## Patentansprüche

1. Inaktivierungsverfahren von Pathogenen, insbesondere von Viren und Bakterien einer Bluttplättchen enthaltenden biologischen Flüssigkeit, wobei das genannte Verfahren die Stufen enthält, die Folgendes vorsehen:
- Vorbereiten und / oder Anordnen der Flüssigkeit in einem Behälter, von dem wenigstens ein Teil der Wände deutlich transparent für die Strahlungen in einer zwischen 350 nm und 450 nm inbegriffenen Strahlenlänge ist;
- Einführen der 5-Aminolävulinsäure (ALA) oder einer ihrer Derivate in die Flüssigkeit derart, dass die Umwandlung von ALA in Protoporphyrin IX zugelassen wird;
- Beleuchten des Behälters mit wenigstens einer Strahlung einer zwischen 350 nm und 450 nm inbegriffenen Wellenlänge derart, dass das Protoporphyrin IX aktiviert wird und die Pathogene inaktiviert werden;
wobei das genannte Verfahren **dadurch gekennzeichnet ist, dass** vor dem Einführen der ALA die Flüssigkeit deleukozytiert wird.

2. Verfahren gemäß Anspruch 1, **dadurch gekennzeichnet, dass** die biologische Flüssigkeit ein Plättchenkonzentrat ist.

3. Verfahren gemäß Anspruch 1 oder 2, **dadurch gekennzeichnet, dass** die ALA vor ihrer Einführung in die Flüssigkeit gelöst wird, wobei die genannte Lösung in den Behälter eingeführt wird.

4. Verfahren gemäß Anspruch 1 bis 3, **dadurch gekennzeichnet, dass** die ALA vor dem Einbringen der Flüssigkeit in den genannten Behälter in den Behälter eingebracht wird.

5. Verfahren gemäß Anspruch 1 bis 3, **dadurch gekennzeichnet, dass** die ALA beim Einbringen der genannten Flüssigkeit in den Behälter in die Flüssigkeit eingebracht wird.

6. Verfahren gemäß Anspruch 1 bis 5, **dadurch gekennzeichnet, dass** die in die Flüssigkeit eingeführte ALA-Konzentration niedriger als oder gleich 5 mM ist.

7. Verfahren gemäß Anspruch 1 bis 6, **dadurch gekennzeichnet, dass** vor der Beleuchtung darüber hinaus ein Eisen-Chelatbildner in die Flüssigkeit eingeführt wird.

8. Verfahren gemäß Anspruch 1 bis 7, **dadurch gekennzeichnet, dass** vor dem Beleuchten die der ALA zugesetzte Flüssigkeit während einer zwischen 0 und 5 Stunden inbegriffenen Zeit inkubiert wird.

9. Verfahren gemäß Anspruch 1 bis 8, **dadurch gekennzeichnet, dass** es bei einer zwischen 0° und 40°C inbegriffenen Temperatur realisiert wird.

10. Verfahren gemäß Anspruch 1 bis 9, **dadurch gekennzeichnet, dass** die Beleuchtung mit einer Bestrahlung mit einer Wellenlänge erfolgt, die im Soret-Band inbegriffen und / oder gleich 365 nm ist.

11. Verfahren gemäß Anspruch 1 bis 10, **dadurch gekennzeichnet, dass** die Beleuchtung während einer zwischen 30 Minuten und 2 Stunden inbegriffenen Zeit mit einer zwischen 10 J/cm² und 100 J/cm² inbegriffenen Energie geliefert wird.
